# EUROPEAN PATENT SPECIFICATION

(11) **EP 1 287 854 B1**
(45) Date of publication and mention of the grant of the patent: **30.11.2005**
(21) Application number: 01307370.5
(22) Date of filing: 30.08.2001
(51) Int. Cl.: A61P 35/00, A61K 31/35

(54) **Anti-cancer combinations of DMXAA and paclitaxel or docetaxel**
Antikrebs-Kombinationen aus DMXAA und Paclitaxel oder Docetaxel
Combinaisons anti-cancéreuses de DMXAA et paclitaxel ou docetaxel

(43) Date of publication of application: 05.03.2003
(73) Proprietor: Cancer Research Technology Limited, London WC2A 3PX (GB)
(72) Inventor: Wilson, William, Robert, RD2 Waiuku (NZ)
(74) Representative: Nachshen, Neil Jacob

(56) References cited:
- WO-A-00/48591
- PATENT ABSTRACTS OF JAPAN vol. 2000, no. 26, 1 July 2002 (2002-07-01) & JP 2001 247459 A (OAKLAND UNISERVICES LTD), 11 September 2001 (2001-09-11) & US 2001/027210 A1 4 October 2001 (2001-10-04)

## Description

The present invention relates to synergistic combinations of the compound 5,6-dimethylxanthenone-4-acetic acid (DMXAA) and paclitaxel or docetaxel, which have anti-tumour activity. More particularly, the invention is concerned with the use of such combinations in the treatment of cancer and pharmaceutical compositions containing such combinations.

5,6-dimethylxanthenone-4-acetic acid (DMXAA) is represented by the following formula:

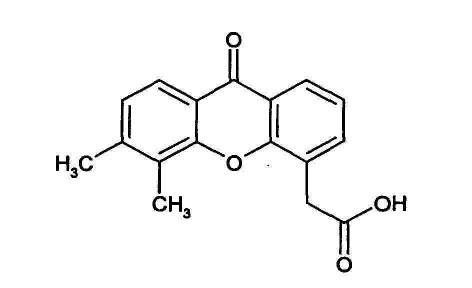

Phase I clinical trials of DMXAA have recently been completed, with dynamic MRI showing that it induces a significant reduction in tumour blood flow at well-tolerated doses. DMXAA is thus one of the first antivascular agents for which activity (irreversible inhibition of tumour blood flow) has been documented in human tumours. These findings are in agreement with preclinical studies using tumours or human tumour xenografts which showed that its antivascular activity produced prolonged inhibition of tumour blood flow leading to extensive regions of haemorrhagic necrosis. However, in such studies tumours rapidly regrow from surviving cells in the well-perfused periphery. The transient tumour growth inhibition seen in most preclinical models is consistent with the lack of tumour regressions seen in the phase I clinical studies, and suggests that DMXAA is unlikely to have clinical utility as a single agent.

Paclitaxel (Taxol®) is a natural product with antitumor activity. Paclitaxel is obtained via a semi-synthetic process from Taxus baccata. The chemical name for paclitaxel is 5β, 20-Epoxy-1,2a,4,7β,10β,13a-hexahydroxytax-11-en-9-one 4,10-diacetate 2- benzoate 13-ester with (2R, 3 S)-N-benzoyl-3-phenylisoserine.

Docetaxel (Taxotere®) is an antineoplastic agent belonging to the taxoid family. It is a semisynthetic product made from an extract of needles of the yew plant. The chemical name for docetaxel is (2R,3S)-N-carboxy-3-phenylisoserine, N *tert*-butyl ester, 13-ester with 5b-20-epoxy-1,2a,4,7b,10b,13a-hexahydroxytax-11-en-9-one 4-acetate 2-benzoate trihydrate.

WO 00/48591 discloses compositions for the treatment of a disease involving active angiogenesis, the compositions comprising a vascular damaging agent (e.g. dimethylxanthenone acetic acid) and an inhibitor of the formation or action of nitric oxide. It is stated that the compositions can be administered as the sole treatment or in combination with other treatments. For example, it is stated that the compositions can be used in combination with other anti-tumour substances such as mitotic agents (e.g. vinblastine, paclitaxel or docetaxel). However, no exemplification of the use of the compositions with such other treatment is provided.

It has now surprisingly been found that by combining, either concomitantly or sequentially, DMXAA with either paclitaxel or docetaxel potentiation of antitumour activity is achieved. In particular, co-administration of DMXAA with paclitaxel or docetaxel provides a therapeutic gain against established (0.5g), early-passage mouse mammary tumours (line MDAH-Mca-4).

Thus, in a first aspect, the present invention provides the use of 5,6-dimethylxanthenone-4-acetic acid (DMXAA) or a pharmaceutically acceptable salt thereof and a compound selected from paclitaxel or docetaxel in the manufacture of a medicament for the treatment of cancer, wherein the 5,6-dimethylxanthenone-4-acetic acid or a pharmaceutically acceptable salt thereof is to be administered either concomitantly or sequentially with said compound selected from paclitaxel or doecetaxel.

In another aspect, the present invention provides a combination of DMXAA or a pharmaceutically acceptable salt thereof and a compound selected from paclitaxel or docetaxel wherein the DMXAA or pharmaceutically acceptable salt thereof and the compound selected from paclitaxel or docetaxel are present in a potentiating ratio.

### Brief Description of the Drawings

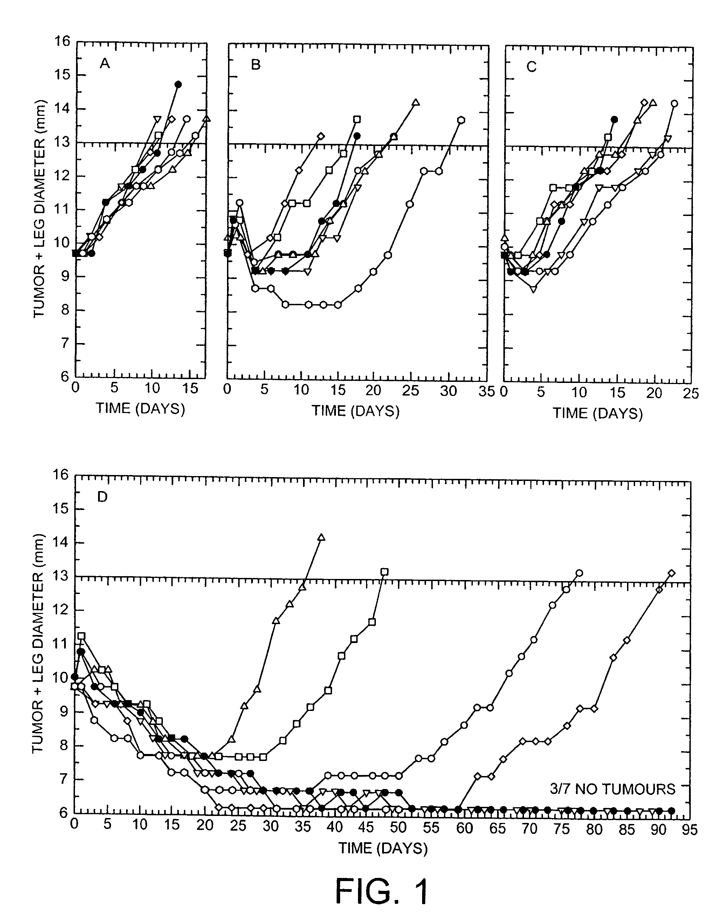

Fig. 1. shows representative individual tumour growth curves for female C₃H/HeN mice to which DMXAA, paclitaxel and DMXAA, and paclitaxel were administered as described in Example 1 herein, as follows: A: controls, B: DMXAA (80 µmol/kg), C: paclitaxel (42.1 µmol/kg), D: DMXAA (80 µmol/kg) + paclitaxel (31.6 µmol/kg).

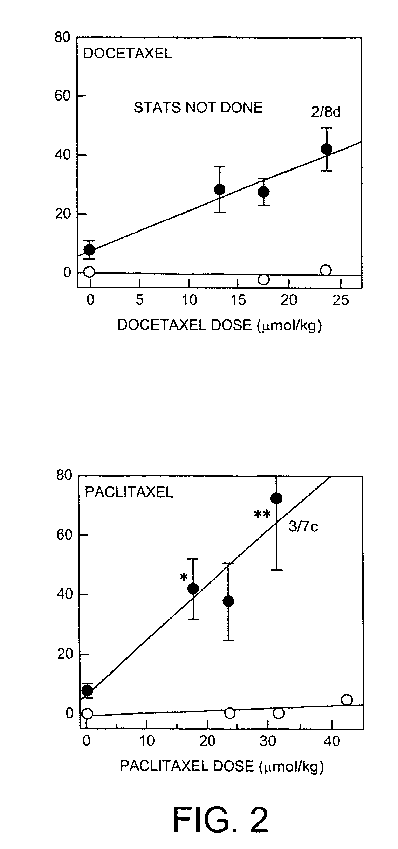

Fig. 2. Growth delay of MDAH-Mca-4 tumours after treatment of mice with DMXAA alone (80 µmol/kg, i.p.), chemotherapeutic drug alone (i.p.), or co-administration of drug with DMXAA (80 µmol/kg). Values are mean ± sem for groups of 6-8 mice, ignoring deaths (d) or cures (c), the numbers of which are shown in parentheses.

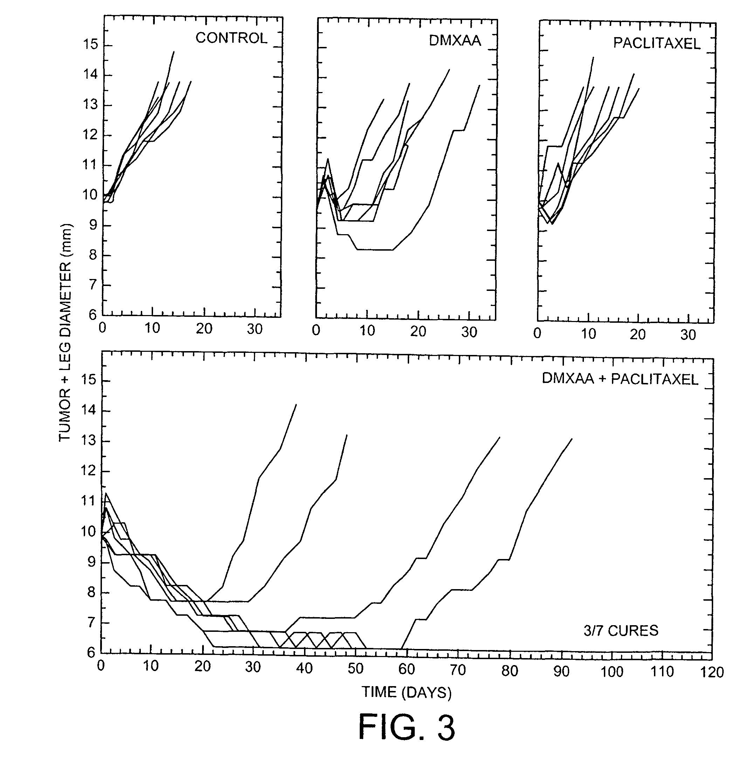

Fig. 3. Individual growth curves for i.m. MDAH-Mca-4 tumours following treatment with DMXAA (80 µmol/kg), paclitaxel (31.6 µmol/kg), or DMXAA plus paclitaxel co-administered.

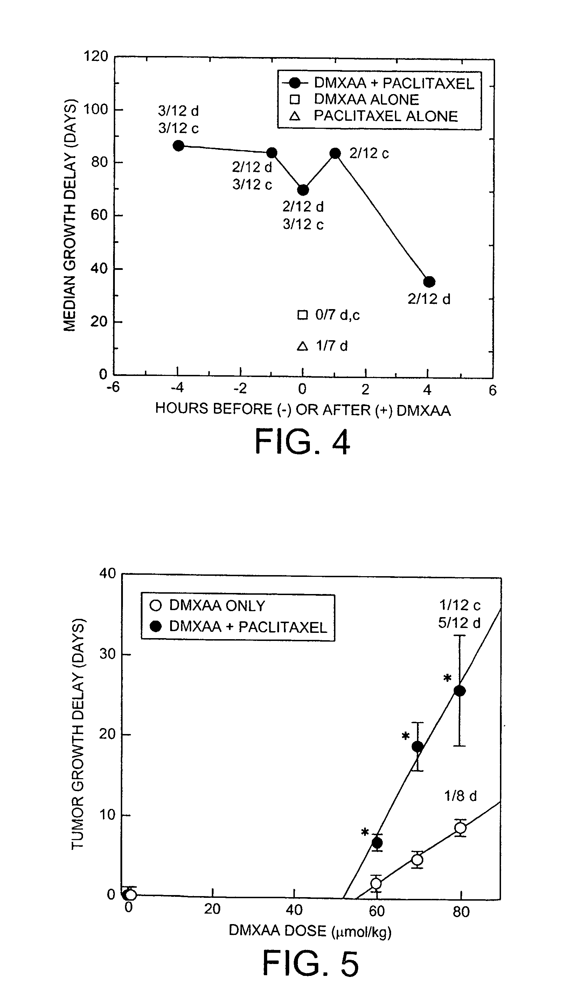

Fig. 4. Effect of varying the timing of paclitaxel (23.7 µmol/kg) relative to DMXAA (80 µmol/kg) on activity against MDAH-Mca-4 tumours. The growth delay is reported as the median for this experiment because of the high proportions of cures (c) and treatment-related deaths (d).
Fig. 5. DMXAA dose dependence of growth inhibition of MDAH-Mca-4 tumours: DMXAA alone or in combination with paclitaxel (23.7 µmol/kg). Values are mean ± sem for 8 (DMXAA only) or 12 (DMXAA + paclitaxel) animals. * indicates activity of the combination significantly greater than DMXAA alone (p<0.05).

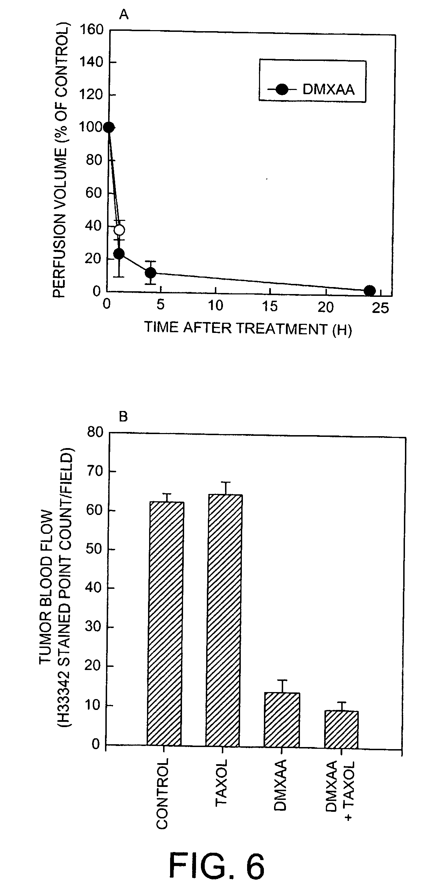

Fig. 6. Antivascular activity of DMXAA against the MDAH-Mca-4 tumour, determined by scoring H33342 perfusion volume by point counting. A: Time course of inhibition of perfusion following DMXAA (80 µmol/kg). Points are mean ± sem. B: Tumour perfusion 4 hr after DMXAA (70 µmol/kg), paclitaxel (23.7 µmol/kg) or co-administration of both drugs. Points are mean ± sem for 5-7 tumours.

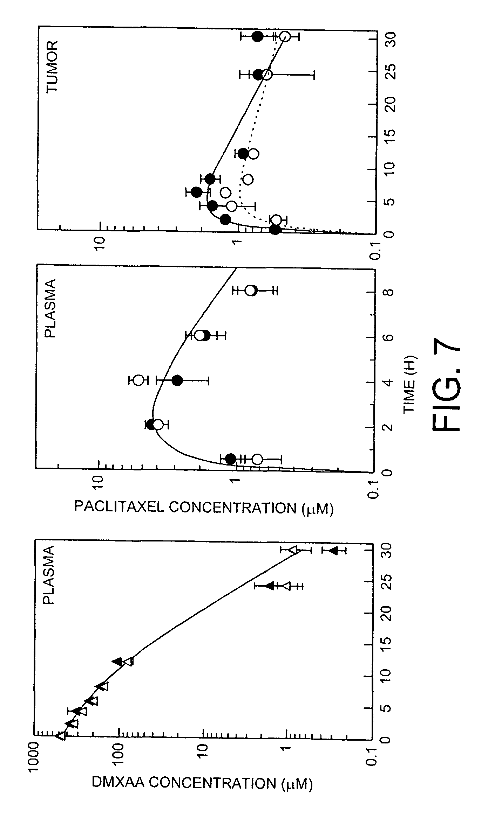

Fig. 7. Left hand panel: Plasma concentrations of DMXAA following administration of DMXAA (80 µmol/kg) alone (filled triangles), or co-administration with paclitaxel (23.7 µmol/kg) (Δ). Centre panel: Plasma concentrations of paclitaxel following administration of paclitaxel alone (●), or co-administered with DMXAA (O). Right hand panel: Tumour concentrations of paclitaxel following administration of paclitaxel alone (●), or co-administration with DMXAA (O). Lines are model fits as described in the text.

The term 'potentiating ratio' is used herein to indicate that the DMXAA or pharmaceutically acceptable salt thereof and the compound selected from paclitaxel or docetaxel are present in a ratio such that the antitumour activity of the combination is greater than that of either DMXAA or paclitaxel or docetaxel alone or of the additive activity that would be predicted for the combinations based on the activities of the individual components. Thus the individual components act synergistically in combination provided they are present in a potentiating ratio.

The DMXAA or pharmaceutically acceptable salt thereof and the compound selected from paclitaxel or docetaxel may be administered concomitantly or sequentially. When the compound selected from paclitaxel or docetaxel is administered first the DMXAA or pharmaceutically acceptable salt thereof may suitably be administered within 4 hours. When the DMXAA or pharmaceutically acceptable salt thereof is administered first the compound selected from paclitaxel or docetaxel may suitably be administered within 2 hours. Preferably the DMXAA or pharmaceutically acceptable salt thereof and the compound selected from paclitaxel or docetaxel are administered within 2 hours of one another. In a preferred embodiment the compound selected from paclitaxel or docetaxel is administered first, followed by administration of the DMXAA or pharmaceutically acceptable salt thereof as soon as practicable, preferably within 4 hours more preferably within 2 hours. Most preferably the DMXAA or pharmaceutically acceptable salt thereof and the compound selected from paclitaxel or docetaxel are administered concomitantly.

The compound selected from paclitaxel or docetaxel is suitably administered intravenously over a period of less than 24 hours, preferably over a period of less than 6 hours more preferably over about 3 hours or less.

The DMXAA or pharmaceutically acceptable salt thereof is suitably administered intravenously over a period of less than 24 hours, preferably over a period of less than 6 hours, more preferably over a period of 3 hours or less. Most preferably the DMXAA or pharmaceutically acceptable salt thereof is administered intravenously over a period of about 1 hour or less.

Optionally, prior to the administration of the compound selected from paclitaxel or docetaxel, pre-medication selected from the group consisting of steroids, antihistamines and H₂ receptor antagonists is given to the patient.

Preferably the DMXAA or pharmaceutically acceptable salt thereof and the compound selected from paclitaxel or docetaxel are administered in a potentiating ratio. Preferably the pharmaceutically acceptable salt is the sodium salt.

A potentiating ratio, for DMXAA and paclitaxel which may be successfully used to treat cancer, is in the range 40:1 to 1:10 of DMXAA:paclitaxel (based on 10 to 200 µmol/kg DMXAA and 5 to 100 µmol/kg of paclitaxel). Suitably, the potentiating ratio is in the range 10:1 to 1:1 (based on 50 to 100 µmol/kg DMXAA and 10 to 50 µmol/kg of paclitaxel). A further potentiating ratio is in the range 6:1 to 4:3 (based on 60 to 90 µmol/kg DMXAA and 15 to 45 µmol/kg of paclitaxel). A preferred potentiating ratio is in the range 19:4 to 15:7 (based on 75 to 85 µmol/kg DMXAA and 20 to 35 µmol/kg of paclitaxel). Further preferred potentiating ratios which may be used with the present invention are in the range 220:1 to 1:1 of DMXAA:paclitaxel, more preferably in the range 60:1 to 1:1 of DMXAA:paclitaxel, more preferably in the range 16:1 to 1:1 of DMXAA:paclitaxel, more preferably in the range 8:1 to 1:1 of DMXAA:paclitaxel, more preferably in the range 4:1 to 1:1 of DMXAA:paclitaxel, more preferably in the range 2:1 to 1:1 of DMXAA:paclitaxel.

A potentiating ratio, for DMXAA and docetaxel which may be successfully used to treat cancer, is in the range 40:1 to 1:10 of DMXAA:docetaxel (based on 10 to 200 µmol/kg DMXAA and 5 to 100 µmol/kg of docetaxel). Suitably, the potentiating ratio is in the range 10:1 to 1:1 (based on 50 to 100 µmol/kg DMXAA and 10 to 50 µmol/kg of docetaxel). A preferred potentiating ratio is in the range 9:1 to 2:1 (based on 60 to 90 µmol/kg DMXAA and 10 to 30 µmol/kg of docetaxel). A particularly preferred potentiating ratio is in the range 17:2 to 3:1 (based on 75 to 85 µmol/kg DMXAA and 10 to 25 µmol/kg of docetaxel). Further preferred potentiating ratios which may be used with the present invention are in the range 100:1 to 1:1 of DMXAA:docetaxel, more preferably in the range 50:1 to 1:1 of DMXAA:docetaxel, more preferably in the range 25:1 to 1:1 of DMXAA:docetaxel, more preferably in the range 10:1 to 1:1 of DMXAA:docetaxel, more preferably in the range 7.5:1 to 1:1 of DMXAA:docetaxel, more preferably in the range 5:1 to 1:1 of DMXAA:docetaxel, more preferably in the range 3.67:1 to 1:1 of DMXAA:docetaxel with the range 2:1 to 1:1of DMXAA:docetaxel being most preferred.

In one embodiment the compound selected from docetaxel or paclitaxel is docetaxel, preferably the compound is paclitaxel.

The amount of a combination of DMXAA or a pharmaceutically acceptable salt thereof and a compound selected from paclitaxel or docetaxel required to be effective as an anticancer agent will, of course, vary and is ultimately at the discretion of the medical practitioner. The factors to be considered include the route of administration and nature of the formulation, the mammal's bodyweight, age and general condition and the nature and severity of the disease to be treated. In general, a suitable effective dose of a combination of DMXAA and paclitaxel for administration to man for treatment of cancer is in the range of 600 to 4900 mg/m² of DMXAA and 15 to 400 mg/m² of paclitaxel. For example from 600 to 4900 mg/m² of DMXAA and 50 to 275 mg/m² of paclitaxel, suitably 1200 to 3500 mg/m² of DMXAA and 50 to 275 mg/m² of paclitaxel, particularly 2000 to 3000 mg/m² of DMXAA and 50 to 275 mg/m² of paclitaxel, particularly 2000 to 3000 mg/m² of DMXAA and 135 to 275 mg/m² of paclitaxel, particularly 2000 to 3000 mg/m² of DMXAA and 135 to 175 mg/m² of paclitaxel, particularly 2000 to 3000 mg/m² of DMXAA and 50 to 135 mg/m² of paclitaxel, more particularly 2250 to 2750 mg/m² of DMXAA and 50 to 275 mg/m² of paclitaxel, more particularly 2250 to 2750 mg/m² of DMXAA and 135 to 275 mg/m² of paclitaxel. A particularly preferred dose is in the range 2250 to 2750 mg/m² of DMXAA and 50 to 135 mg/m² of paclitaxel. A further particularly preferred dose is in the range 2250 to 2750 mg/m² of DMXAA and 135 to 175 mg/m² of paclitaxel.

In general, a suitable effective dose of a combination of DMXAA and docetaxel for administration to man for treatment of cancer is in the range of 600 to 4900 mg/m² of DMXAA and 20 to 200 mg/m² of docetaxel. For example from 600 to 4900 mg/m² of DMXAA and 40 to 120 mg/m² of docetaxel, suitably 1200 to 3500 mg/m² of DMXAA and 40 to 120 mg/m² of docetaxel, more suitably 1200 to 3500 mg/m² of DMXAA and 20 to 60 mg/m² of docetaxel, particularly 2000 to 3000 mg/m² of DMXAA and 40 to 120 mg/m² of docetaxel, particularly 2000 to 3000 mg/m² of DMXAA and 60 to 100 mg/m² of docetaxel, particularly 2000 to 3000 mg/m² of DMXAA and 20 to 60 mg/m² of docetaxel, more particularly 2250 to 2750 mg/m² of DMXAA and 20 to 200 mg/m² of docetaxel, more particularly 2250 to 2750 mg/m² of DMXAA and 40 to 120 mg/m² of docetaxel. A particularly preferred dose is in the range 2250 to 2750 mg/m² of DMXAA and 60 to 100 mg/m² of docetaxel. A further particularly preferred dose is in the range 2250 to 2750 mg/m² of DMXAA and 20 to 60 mg/m² of docetaxel.

The DMXAA or pharmaceutically acceptable salt thereof and the compound selected from paclitaxel or docetaxel may be administered in any suitable form. However, for use according to the present invention the combination of DMXAA or a pharmaceutically acceptable salt thereof and a compound selected from paclitaxel or docetaxel is preferably presented as a pharmaceutical formulation.

Pharmaceutical formulations comprise the active ingredients (that is, the combination of DMXAA or a pharmaceutically acceptable salt thereof and a compound selected from paclitaxel or docetaxel) together with one or more pharmaceutically acceptable carriers therefor and optionally other therapeutic and/or prophylactic ingredients. The carrier(s) must be acceptable in the sense of being compatible with the other ingredients of the formula and not deleterious to the recipient thereof.

Accordingly, the present invention provides a pharmaceutical formulation comprising a combination of DMXAA or a pharmaceutically acceptable salt thereof and a compound selected from paclitaxel or docetaxel in association with one or more pharmaceutically acceptable carriers therefor.

The present invention further provides a process for the preparation of a pharmaceutical formulation which process comprises bringing into association a combination of DMXAA or a pharmaceutically acceptable salt thereof and a compound selected from paclitaxel or docetaxel with one or more pharmaceutically acceptable carriers therefor.

Pharmaceutical formulations include those suitable for oral, topical (including dermal, buccal and sublingual), rectal and parenteral (including subcutaneous, intradermal, intramuscular and intravenous) administration as well as administration by naso-gastric tube. The formulation may, where appropriate, be conveniently presented in discrete dosage units and may be prepared by any of the methods well known in the art of pharmacy. All methods include the step of bringing into association the active ingredients with liquid carriers or finely divided solid carriers or both and then, if necessary, shaping the product into the desired formulation.

Preferably the pharmaceutical formulations is adapted for parenteral administration, most preferably intravenous administration. For example the compounds may be administered intravenously using formulations for each compound already known in the art.

Pharmaceutical formulations suitable for oral administration wherein the carrier is a solid are most preferably presented as unit dose formulations such as boluses, capsules or tablets each containing a predetermined amount of the active ingredients. A tablet may be made by compression or moulding, optionally with one or more accessory ingredients. Compressed tablets may be prepared by compressing in a suitable machine the active compounds in a free-flowing form such as a powder or granules optionally mixed with a binder, lubricant, inert diluent, lubricating agent, surface-active agent or dispersing agent. Moulded tablets may be made by moulding an inert liquid diluent. Tablets may be optionally coated and, if uncoated, may optionally be scored. Capsules may be prepared by filling the active ingredients, either alone or in admixture with one or more accessory ingredients, into the capsule shells and then sealing them in the usual manner. Cachets are analogous to capsules wherein the active ingredients together with any accessory ingredient(s) are sealed in a rice paper envelope. The combination of DMXAA or a pharmaceutically acceptable salt thereof and a compound selected from paclitaxel or docetaxel may also be formulated as dispersible granules, which may for example be suspended in water before administration, or sprinkled on food. The granules may be packaged e.g. in a sachet. Formulations suitable for oral administration wherein the carrier is a liquid may be presented as a solution or a suspension in an aqueous liquid or a non-aqueous liquid, or as an oil-in-water liquid emulsion.

Formulations for oral administration include controlled release dosage forms e.g. tablets wherein the active ingredients are formulated in an appropriate release - controlling matrix, or are coated with a suitable release - controlling film. Such formulations may be particularly convenient for prophylactic use.

The active ingredients may also be formulated as a solution or suspension suitable for administration via a naso-gastric tube.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the active combination with the softened or melted carrier(s) followed by chilling and shaping in moulds.

Pharmaceutical formulations suitable for parenteral administration include sterile solutions or suspensions of the active combination in aqueous or oleaginous vehicles. Injectible preparations may be adapted for bolus injection or continuous infusion. Such preparations are conveniently presented in unit dose or multi-dose containers which are sealed after introduction of the formulation until required for use. Alternatively, the active ingredients may be in powder form which are constituted with a suitable vehicle, such as sterile, pyrogen-free water, before use.

The combination of DMXAA or a pharmaceutically acceptable salt thereof and compound selected from paclitaxel or docetaxel may also be formulated as a long-acting depot preparation, which may be administered by intramuscular injection or by implantation e.g. subcutaneously or intramuscularly. Depot preparations may include, for example, suitable polymeric or hydrophobic materials, or ion-exchange resins. Such long-acting formulations are particularly convenient for prophylactic use.

It should be understood that in addition to the aforementioned carrier ingredients the pharmaceutical formulations for the various routes of administration described above may include, as appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

DMXAA may be prepared according to the methods described in Journal of Medicinal Chemistry 34(1): 217-22, January 1991 the contents of which are incorporated herein by reference.

The taxanes paclitaxel (Taxol®) and docetaxel (Taxotere®) are well known compounds and can be prepared by methods known to those skilled in the art.

It is to be understood that the present invention covers all combinations of suitable and preferred groups described hereinabove.

The present invention will now be illustrated by means of the following examples.

### EXAMPLES

### Example 1

Murine mammary carcinoma MDAH-Mca-4 tumours were grown in female C₃H/HeN mice from stocks stored in liquid nitrogen at the sixth transplant generation, and were passaged and used for experiments at the eighth transplant generation. Tumours for experiments were grown by inoculating 20 µl of cell suspension (5 mg packed cells) intramuscularly in the right gastrocenmius muscle. Animals were randomised to treatment groups, and treated with drugs when the diameter of the tumour-bearing leg reached 10-11 mm (ca. 0.6 g tumour), approximately 18 days after inoculation. Drugs were administered once only, by intraperitoneal injection in phosphate buffered saline (DMXAA) or chremophor (paclitaxel) using volumes of 0.01 ml/g body weight for each compound. Both compounds were administered essentially at the same time (within 1 min). Host toxicity was assessed by determination of lethality, and by measurement of body weight 4-5 days after drug treatment. The diameter of the tumour-bearing leg was measured three times weekly until values exceeded 13 mm (ca 1.4 g tumour) at which time the animals were terminated. The growth curve for each individual tumour was plotted as in Figure 1, and the time-to-endpoint (TTE, time from treatment to endpoint) was determined for each mouse. The median and mean TTE was determined for each treatment group. The mean tumour growth delay for each group was calculated as the difference between mean TTE for treated and control groups. The statistical significance of the antitumour effects was tested using ANOVA followed by Dunnett's test to determine p values for the significance of differences between individual groups.

The data in Figure 1 and Table 1 show that a clear increase in toxicity was seen in the combination, requiring lowering of dose from 42 µmol/kg (not demonstrably toxic for paclitaxel alone) to 31.6 µmol/kg when combined with DMXAA. All mice survived in good health when the latter dose was combined with DMXAA (80 µmol/kg), with no greater weight loss than DMXAA alone. Paclitaxel alone provided little antitumour activity in this model (growth delay of 4 days at 42 µmol/kg), but activity was dramatically enhanced in the combination. Combining taxol at 31.6 µmol/kg with DMXAA provided 3/7 cures, with a mean growth delay of 50 days (42 days longer than DMXAA alone) for the 4 tumours that recurred. Large growth delays were also seen in combination with DMXAA at the two lower taxol dose levels investigated, with one cure at 17.8 µmol/kg.

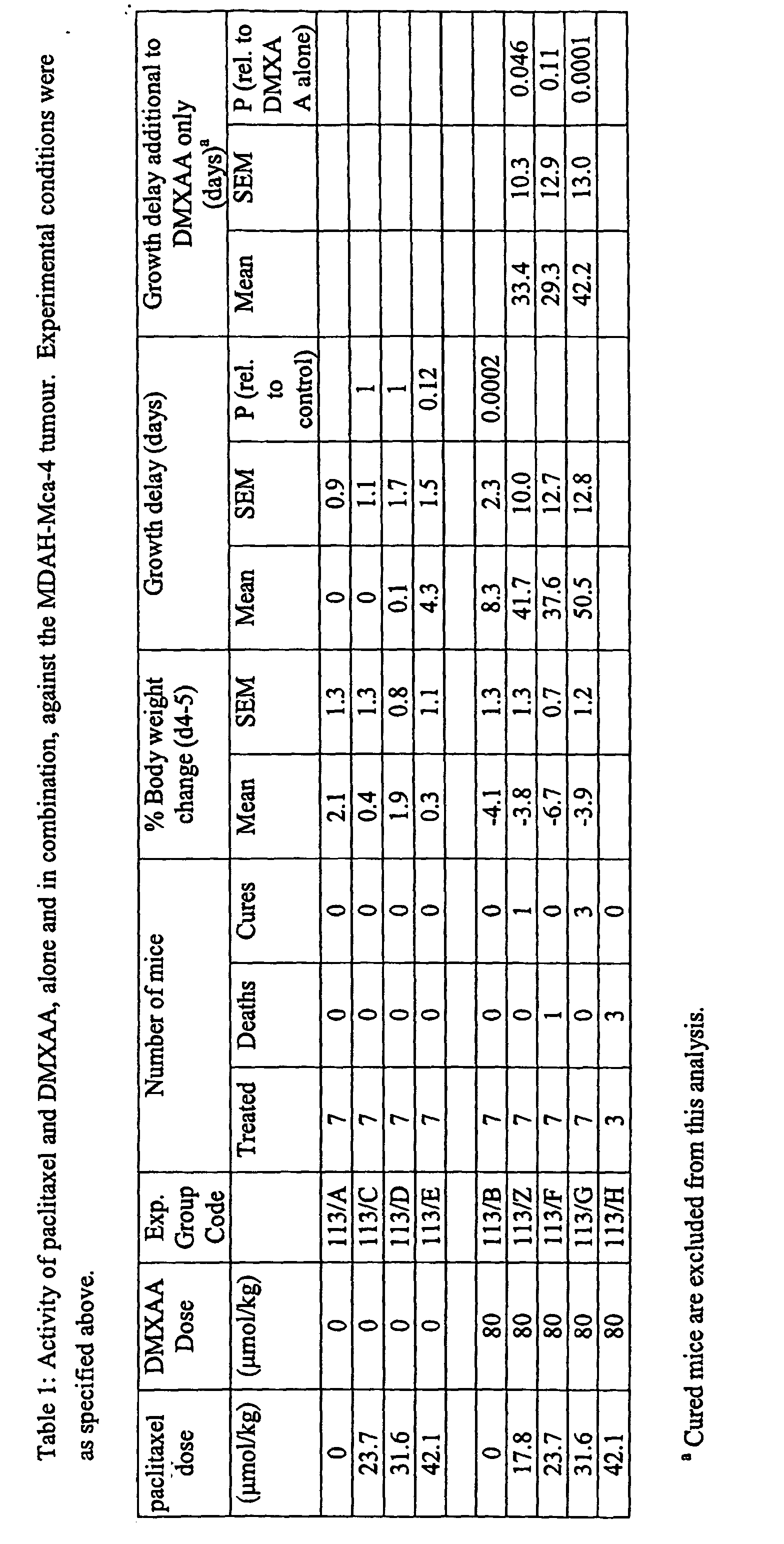

### Example 2

The effect of varying the time of administration of DMXAA relative to paclitaxel was investigated using the same experimental approach as in Example 1. This analysis based on the median time for tumours to reach the endpoint size (Table 2) confirms the large interaction demonstrated in Example 1. At this time it is not possible to discriminate the magnitude of the response for the groups treated with DMXAA 1 hr before paclitaxel or 1 hr after paclitaxel or 4 hr after paclitaxel or co-administration. The response is not as large with DMXAA administered 4 hr before paclitaxel. This result demonstrates a reasonably broad time window for the interaction.

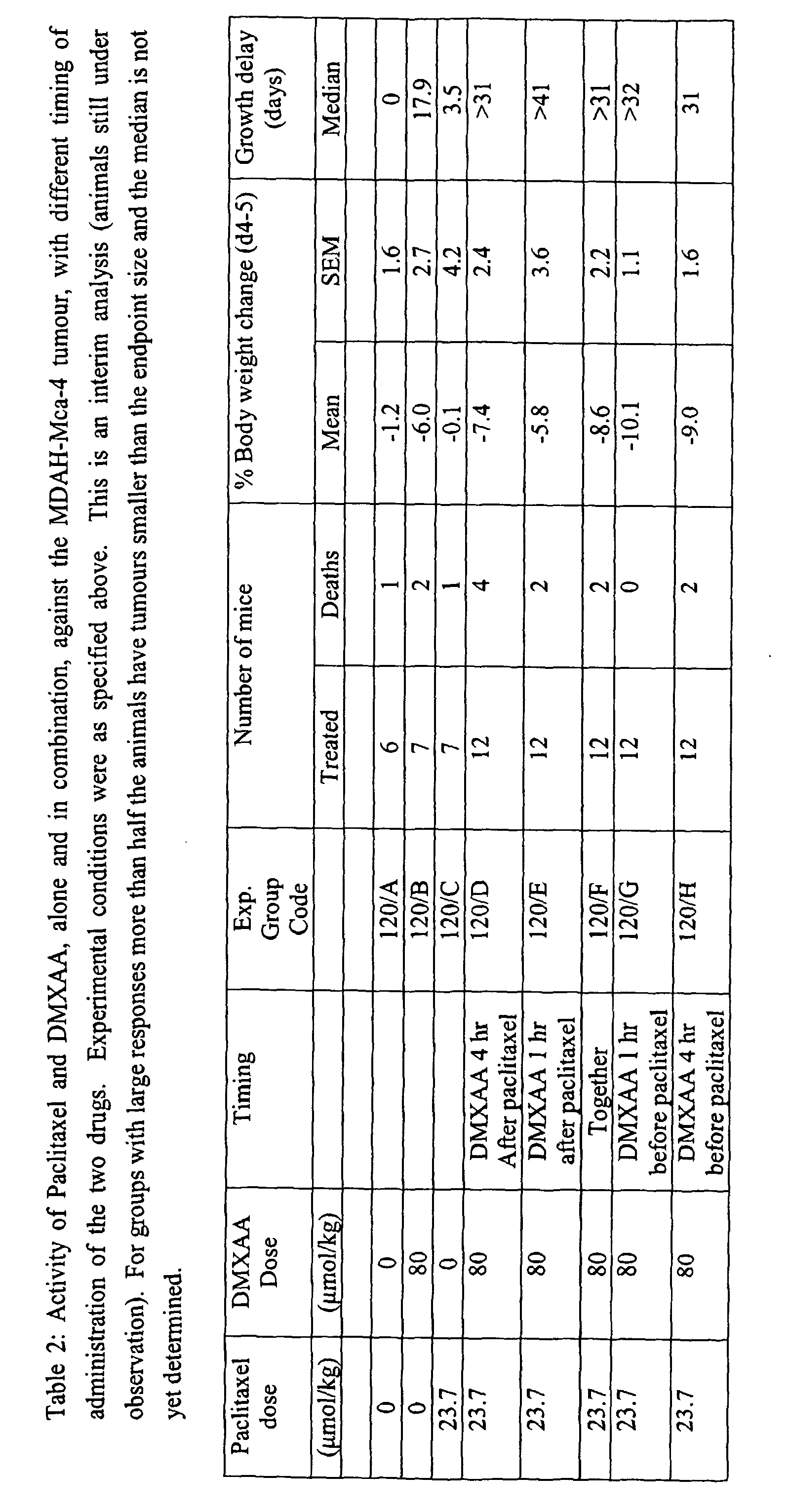

### Example 3

The interaction of docetaxel with DMXAA was investigated using the same experimental approach as in Example 1, with co-administration of both compounds in the combination. This analysis (based on the median time for tumours to reach the endpoint size (Table 3) indicates that docetaxel alone is essentially devoid of activity against the MDAH-Mca-4 tumour, but that the combination of docetaxel and DMXAA is much more active than DMXAA alone. Thus, as for paclitaxel, the activity of the combination is much greater than would be expected on the basis of the activities of the single agents.

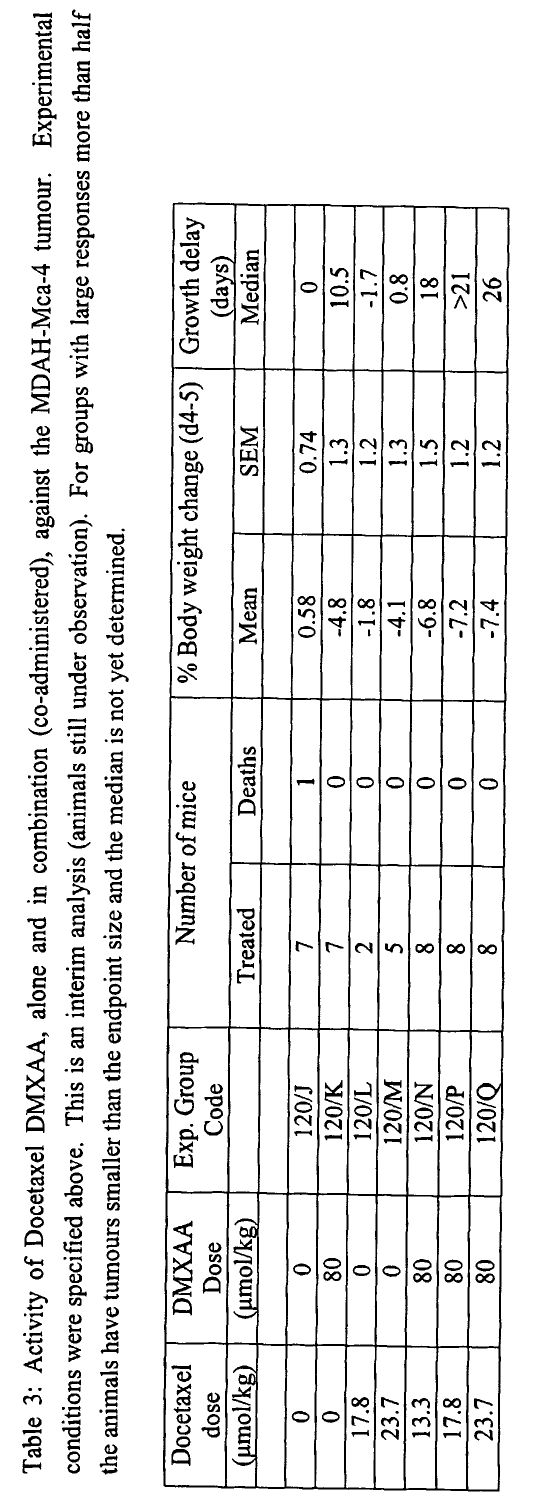

### FURTHER EXAMPLES

### Materials And Methods

Compounds: A stock solution of DMXAA, synthesised in the Auckland Cancer Society Research Centre, was prepared in phosphate-buffered saline, protected from light and stored frozen. Docetaxel was diluted using 0.9% saline. Paclitaxel was stored frozen in ethanol, and diluted sequentially with Cremophor EL (1 vol) and 0.9% saline (10 vols) immediately before use. All compounds were administered to mice by i.p. injection at 0.01 ml/g body weight.

Animals and Tumours: Murine mammary carcinoma MDAH-Mca-4 tumours were grown from stocks stored in liquid nitrogen at the sixth transplant generation. Tumours (eighth transplant generation when used) were grown from 20 µl of cell suspension (7 mg packed cells), inoculated i.m. in the right gastrocnemius muscle of female C₃H/HeN mice (22-25 g at the time of treatment). Mice were randomised to treatment which commenced when the tumour + leg diameter reached 10-11 mm (0.5-0.7 g tumour).

Host Toxicity and Antitumour Activity: Mice were treated with chemotherapeutic drugs at a range of doses, at 1.33-fold increments, up to the expected MTD (maximum tolerated dose, as estimated in pilot experiments or from the literature). Toxicity was assessed as lethality, and body weight loss measured four days after treatment. Any animals becoming moribund were terminated and treated as drug-related deaths in the analysis. The diameter of the tumour-bearing leg was measured 3 times per week after treatment. Antitumour activity was assessed from the tumour growth delay, defined as the difference in time to reach the endpoint of 13 mm (1.5 g tumour) for treated and control groups. Responses were classed as cures if animals were free of evident tumour 120 days after treatment. The statistical significance of tumour growth inhibition was tested by ANOVA using SAS for Windows, with Dunnett's test to evaluate *p*-values for differences between treatment groups. In experiments with substantial numbers of cures (free of tumour for > 120 days after treatment), statistical significance was tested by Kruskal-Wallis non-parametric analysis of variance using SAS for Windows, and the difference between treatment and control groups by Dunn's test using Sigmastat v2.0. The gradient and standard error of dose-response curves was determined by linear regression using Sigmastat, and the DMF calculated as the gradient with DMXAA/gradient without DMXAA.

Tumour blood flow inhibition: Tumour blood flow was assessed using the fluorescent perfusion marker Hoechst 33342 (8mg/ml in saline), which was administered i.v. at various times after drug treatment. Mice were scarified 2 min later, and frozen sections (14 µm) prepared from the distal, central and proximal regions of each tumour. Sections were examined with a Nikon epifluorescence microscope at 10x magnification using a UV-1A filter block (excitation 365 nm, barrier filter 400 nm, and dichroic mirror 400 nm). A grid with 81 squares (100 x 100 µm), was used for point scoring of staining. The whole area of each section was scored to avoid bias between peripheral and central regions (which were less well perfused). Normal tissue was excluded but necrotic areas were included. Differences between groups were treated for significance using the Student's *t*-test (Sigma Stat, version-2.0; Jandel Scientific Limited).

Pharmacokinetics: Female C₃H/HeN mice bearing MDAH-Mca-4 tumours (0.5-0.7 g) were injected i.p. with paclitaxel (23.7 µmol/kg), DMXAA (80 µmol/kg), or simultaneously received paclitaxel and DMXAA, at the same doses. At various times blood was collected from the retro-orbital sinus of anaesthetised mice into heparinised tubes, and the plasma separated by centrifugation. Tumours were rapidly dissected and frozen at -80°C. Groups of 2-5 mice were used for each time point.

LC-MS analysis of paclitaxel: Concentrations of paclitaxel in plasma and tumour were determined by HPLC with detection by positive mode electrospray mass spectrometry. Aliquots of plasma (50 µl) were treated with 1ml of ice-cold acetonitrile:methanol (3:1 v/v) to precipitate proteins. Following addition of 5 µl of an internal standard solution (10 mM cephalomannine), samples were centrifuged, and the resulting supernatants evaporated using a Speed-Vac solvent concentrator (Savant Instruments, NY). The residues were dissolved in 100 µl of 1:1 (v/v) methanol:mobile phase (48% acetonitrile, 52% 1mM sodium acetate buffer (pH 6.2)), and 5-40 µl analysed by LC-MS. Frozen tumours were minced, then homogenised in 2 vol distilled water using a Polytron homogeniser. Ice-cold acetonitrile (1 ml), and 10 µl of internal standard (20 µM cephalomannine) was added to 200 µl of tumour homogenate. The samples were centrifuged and the supernatants dried as above. The residues were dissolved in 200 µl of 1:1 (v/v) methanol:mobile phase, and 5 µl analysed by LC-MS.

The LC-MS system was an Agilent HP1100/MSD (Hewlett Packard Waldbronn, Germany) with a 3.2 mm x 150 mm C₈ 5 µm column (Alltima Associates Inc., Deerfield, IL) and a flow rate of 0.7 ml/min. The mass detector used N₂ as the nebulizing and drying gas (flow rate 10 L/min, 350°C; neubulizer pressure 25 psi, capillary voltage 2500 V, fragmentor voltage 120 V). Paclitaxel (MW 854) and cephalomannine internal standard (MW 832) were detected, using single ion monitoring, as sodium adducts at m/z of 876 and 854, respectively. Spiking of control material indicated assay linearity from 0.3-30 µM for both plasma and tumour (r² = 0.999). The intra- and inter- assay precision and accuracy gave coefficients of variation ≤ 5%, and recoveries in the range 92-102%. The lower limit of detection of paclitaxel in plasma and tumour (signal:noise ratio of 3) was 0.3 µM.

HPLC analysis of DMXAA: Concentrations of DMXAA in plasma were determined as follows. Aliquots of plasma (50 µl) were treated with 1 ml of ice-cold acetonitrile:methanol (3:1 v/v), centrifuged, and the resulting supernatants evaporated as above. The residues were dissolved in 200 µl of 10 mM ammonium acetate buffer (pH 5) and 25 µl was analysed by HPLC using a HP1100 system with diode array detector (278 nm) and fluorescence detector. The column and flow rate were as for paclitaxel above, with a mobile phase of 16% acetonitrile (v/v) in 10 mM ammonium acetate buffer (pH 5). Spiking of control plasma showed assay linearity from 0.1-100 µM (r² = 0.999). The intra- and inter- assay precision and accuracy gave coefficients of variation <7%, and an average recovery of 70%. The lower sensitivity limit of detection by fluorescence (signal:noise ration of 3) was 0.1 µM.

Pharmacokinetic Modelling: Modelling of pharmacokinetic data was done using ModelMaker version 4.0 (Cherwell Scientific Limited, The Magdalen Centre, Oxford Science Park, Oxford OX4 4GA, United Kingdom). The following pharmacokinetic parameters were used: K_{abs}, the first-order rate constant for absorption into the central compartment; Cl, total body clearance; Clᵢₙₜₑᵣ, intercompartmental clearance; V_{d}, apparent volume of distribution of the central compartment; V_{d2}, apparent volume of distribution of the second compartment; Kₘ, Michaelis-Menten constant; Vₘₐₓ, theoretical maximum rate; AUC, area under the concentration-time curve. For all compounds it was assumed that all of the administered dose would reach the central compartment (i.e. 100% bioavailability). Differences between treatment groups were tested using a F-test comparing the entire curves and if this difference was significant (i.e. p≤0.05) the estimates of each individual model parameter for both groups were tested using a 2-tailed t-test. The concentrations of paclitaxel in plasma and tumour were fitted with a 1-compartment open model assuming linear pharmacokinetics (a 2-compartment model for the tumour data was rejected based on a F-test). Plasma concentrations of DMXAA were fitted using a 1-compartment open model with saturable (Michaelis-Menten) elimination kinetics.

### Results

Activity of DMXAA + chemotherapy drugs against MDAH-Mca-4 tumour: The antitumour activity and host toxicity of DMXAA/cytotoxic drug combinations was assessed by varying the dose of chemotherapeutic drug up to the toxicity limit, with co-administration of a fixed DMXAA dose (80 µmol/kg, ca. 80% of MTD), and evaluating subsequent tumour growth delay. Docetaxel and paclitaxel were essentially inactive, as indicated by dose-response relationships providing insignificant slopes by linear regression (Fig 2), and highly significant growth delays of ca 10 days at their MTDs (recorded in Table 4), with no individual treatment groups showing significant activity (although and paclitaxel gave weakly positive dose responses by linear regression).

DMXAA alone showed appreciable activity as a single agent at 80 µmol/kg, providing transient regressions and mean tumour growth delays in the range 3.5 - 8.3 days (overall mean 6.6 ± 0.6 days). Co-administration of DMXAA at this dose increased the host toxicity of docetaxel and paclitaxel; in each case the MTD for the chemotherapy drug was lowered by one dose level (1.33-fold) in the combination (Table 4).

In contrast to this small effect on host toxicity, co-administration of DMXAA produced a large enhancement of tumour growth delay for docetaxel and paclitaxel (Fig 2 and Table 4). The contribution of DMXAA was assessed by determining the slope of each dose-response curve by linear regression, and the DMF for DMXAA was calculated as the ratio of slopes with and without DMXAA. By this criterion, both paclitaxel and docetaxel showed significant synergy, with the DMF significantly greater than unity. As an alternative criterion, the maximum tumour growth delay achievable at the MTD of the combination again indicated both taxanes (paclitaxel and docetaxel) to be active compounds in combination with DMXAA Individual tumour growth curves are illustrated for the experiment in which paclitaxel was combined with DMXAA (Fig 3), demonstrating that the antivascular agent alone causes transient regressions while the combination causes variable but marked responses, including 3/7 complete cures.

DMXAA + paclitaxel, timing and dose-response relationship: The interaction between DMXAA and paclitaxel was examined in further detail. Repetition of the experiment illustrated in Fig 2 confirmed the dramatic activity of this combination with 3/7 cures at a paclitaxel dose of 23.7 µmol/kg and 1/7 cure at 31.6 µmol/kg. One drug-related death was observed in each of these groups, but body weight loss was greater at the higher dose (4.3 ± 1.8 % versus 7.4 ± 1.8 % respectively). The lower paclitaxel dose (23.7 µmol/kg) was used in all further studies.

The time of administration of the two agents was varied, with paclitaxel (23.7 µmol/kg) administered up to 4 hr before or after DMXAA (Fig 4). Substantial activity was seen in most groups, with 2-3 cures per group of 12 mice and large tumour growth delays (median ca. 80 days) for the recurring tumours. The exception was paclitaxel 4 hr after DMXAA which provided no cures and a smaller growth delay which, because of the large variability, was below the level of statistical significance. In each group (including co-administration) the toxicity of the combination was higher than in the previous co-administration experiments, with 2-3 deaths/12 mice and mean body weight loss in the range 6-10%.

Given the appreciable toxicity of the DMXAA/paclitaxel combinations, the DMXAA dose-response relationship was also examined (Fig 5). As in the other experiments, paclitaxel alone at 23.7 µmol/kg had no significant activity, but was significantly enhanced by DMXAA at all three dose levels tested. DMXAA showed a steep dose-response relationship in the combination, with a threshold at a DMXAA dose of approximately 55 µmol/kg. In this experiment the combination of paclitaxel (23.7 µmol/kg) with DMXAA at 80 µmol/kg was highly toxic (5/12 deaths) with 1 cure from the 7 remaining animals, although body weight loss (mean 8 ± 3 %) was in the usual range for this dose level and was no higher than for DMXAA alone (8 ± 2%).

Antivascular activity of DMXAA and effect of paclitaxel co-administration: The abilities of DMXAA a to inhibit blood flow in the MDAH-Mca-4 tumour were compared using H33342 as a fluorescent perfusion marker (Fig 6A). DMXAA at 80 µmol/kg caused prompt and irreversible blood flow shutdown, with no recovery by 24 hr. Whether paclitaxel enhanced the vascular shutdown by DMXAA at 24 hr was assessed in a separate experiment, using a lower DMXAA dose (70 µmol/kg) so that any increased activity could be more readily detected (Fig 6B). This showed no antivascular activity for paclitaxel alone, and no significant increase in activity for the combination relative to DMXAA alone.

Pharmacokinetics of DMXAA and paclitaxel: Studies were conducted to deduce whether a pharmacokinetic interaction underlies the synergistic therapeutic interaction between DMXAA and paclitaxel. The study was conducted in C₃H mice bearing MDAH-Mca-4 tumours of the same size as in the therapeutic studies. The pharmacokinetics of both agents, paclitaxel and DMXAA, alone and after co-administration were compared (Fig 7). DMXAA was measured by HPLC using fluorescence detection (Fig 7A), and paclitaxel by LC-MS (Fig 7B,C). The latter employed electrospray mass spectrometry detection with a high sodium ion concentration in the mobile phase to suppress fragmentation, providing the [M+Na]⁺ molecular ion. DMXAA clearance from plasma was fitted using the previously-reported 1-compartment open model with saturable (Michaelis-Menten) elimination kinetics, providing the pharmacokinetic parameters in Table 6; these were unaffected by co-administration of paclitaxel at 23.7 µmol/kg. Similarly, DMXAA co-administration had no effect on plasma concentrations of paclitaxel (Fig 7B); the latter data were fitted assuming a linear 1-compartment open model, the parameters of which (Table 6) were not significantly different with or without DMXAA. Paclitaxel concentrations in tumours at early times appeared to be slightly lowered by DMXAA co-administration, which led to a higher estimate for the apparent volume of distribution of paclitaxel in the pharmacokinetic model (Table 6). This change in paclitaxel concentrations in tumours is in the wrong direction to account for the enhanced antitumour activity of the combination.

This study tests the hypothesis that antivascular agents such as DMXAA have the potential to combine synergistically with paclitaxel and docetaxel in the treatment of solid tumours. In the early passage mammary tumour MDAH-Mca-4 used for this study, DMXAA alone showed consistent activity as a single agent but did not provide prolonged regressions or cures. However, co-administration of DMXAA with paclitaxel and docetaxel caused a marked increase in response (Fig 2). This interaction can be classified as a synergistic (super-additive) on the basis of the increased slope of the cytotoxic dose response curve on addition of DMXAA. The interaction, quantified as the DMF (ratio of the linear regression slopes with an without DMXAA), was significantly greater than unity.

**Table 4**

| Effect of DMXAA on host toxicity and antitumour activity of chemotherapeutic drugs against MDAH-Mca-4 tumours. Drugs were co-administered with DMXAA by i.p. injection | | | | | |
|---|---|---|---|---|---|
| Chemotherapy drug | DMXAA (µmol/kg) | MTD (µmol/kg) | % body weight change at 4 days | Slope of dose/response (days/ µmol/kg) | DMF |
| Docetaxel | - | 23.7 | -4.1 ± 1.3 | -0.02 ± 0.14 | >9^{c} |
| | 80 | 17.8 | -7.2 ± 1.2 | 1.37 ± 0.22 | |
| Paclitaxel | - | 42.1 | 0.3 ± 1.1 | 0.08 ± 0.06 | >13^{c} |
| | 80 | 31.6 | -3.9 ± 1.2 | 1.83 ± 0.4 | |

| | | | | | |
|---|---|---|---|---|---|
| ^{c}Estimated using upper error estimate of the slope for the chemotherapy drug only. | | | | | |

**Table 5.**

| Comparison of DMXAA (80 µmol/kg) alone and in combination with paclitaxel (23.7 µmol/kg) against the MDAH-Mca-4 tumour. | | | | | | |
|---|---|---|---|---|---|---|
| Treatment | % weight change d4 | Deaths | Cures | Tumour growth delay (days) | | p^{a} |
| | Mean ± sem | | | Median | Mean ± sem | |
| Control^{b} | 0.8 ± 1.7 | 1/7 | 0/7 | 0 | 0.0 ± 0.9 | - |
| Paclitaxel | 2.4 ± 1.0 | 0/7 | 0/7 | -0.3 | -0.1 ± 0.9 | ns |
| DMXAA | -8.1 ± 2.6 | 1/7 | 1/7 | 10.1 | 15.0 ± 3.5 | ∗∗∗∗ |
| DMXAA + Paclitaxel | -2.7 ± 0.5 | 4/11 | 4/11 | 29.5 | 29.0 ± 2.4 | ∗∗∗∗ |

| | | | | | | |
|---|---|---|---|---|---|---|
| ^{a} Time to endpoint 9.4 ± 0.9 days | | | | | | |

**Table 6.**

| Pharmacokinetic parameters for paclitaxel (23.7 µmol/kg), and DMXAA (80 µmol/kg) in plasma and tumour of female C₃H/HeN mice bearing MDAH-Mca-4 tumours (ca 0.7g). Numbers in parentheses are % CV. | | | |
|---|---|---|---|
| Parameter | Plasma | | Tumour |
| | Paclitaxel | DMXAA | Paclitaxel |
| K_{abs} (hr⁻¹) | 0.41 (266) | 9.3 (39) | 0.47 (33) |
| Kₘ | - | 220 (9.2) | - |
| Vₘₐₓ (µM hr⁻¹) | - | 63 (6.1) | - |
| Cl (1 hr⁻¹ kg⁻¹) | 0.85 (14) | - | 0.57 (19) |
| Clᵢₙₜₑᵣ (1 hr⁻¹ kg⁻¹) | - | - | - |
| V_{d} 1 kg⁻¹ | 2.1 (257) | 0.17 (1.2) | 9.5 (16) |
| V_{d} (1 kg⁻¹) | - | - | 19.0 (18)^{c} |
| (coadmin.) | | | |
| V_{d2} (1 kg⁻¹) | - | - | - |
| AUC (µM.hr)^{a} | 18.8^{d} | 3628^{c} | 32.8^{c} |
| AUC (µM.hr)^{a} | 23.2^{c,d} | 3136^{c,f} | 22.3^{c,e} |
| (coadmin.) | | | |

| | | | |
|---|---|---|---|
| ^{a} AUC was calculated using the linear trapezoidal rule ^{b} 0-24 hours | | | |
| ^{c} Coadministration with DMXAA | | | |
| ^{d} 0-8 hours | | | |
| ^{e} 0-30 hours | | | |
| ^{f} Coadministration with paclitaxel | | | |

## Claims

1. Use of 5,6-dimethylxanthenone-4-acetic acid (DMXAA) or a pharmaceutically acceptable salt thereof and a compound selected from paclitaxel or docetaxel in the manufacture of a medicament for the treatment of cancer, wherein the 5,6-dimethylxanthenone-4-acetic acid or a pharmaceutically acceptable salt thereof is to be administered either concomitantly or sequentially with said compound selected from paclitaxel or doecetaxel.

2. Use according to claim 1 wherein the DMXAA or pharmaceutically acceptable salt thereof and the compound selected from paclitaxel or docetaxel are present in a potentiating ratio.

3. Use according to claim 1 or claim 2 wherein the compound selected from paclitaxel or docetaxel is paclitaxel.

4. Use according to claim 3, wherein the ratio of DMXAA: paclitaxel is in the range 220:1 to 1:1.

5. Use according to claim 4 wherein the ratio of DMXAA: paclitaxel is in the range 2:1 to 1:1.

6. Use according to claim 1 or claim 2 wherein the compound selected from paclitaxel or docetaxel is docetaxel.

7. Use according to claim 6 wherein the ratio of DMXAA:docetaxel is in the range 100:1 to 1:1.

8. Use according to claim 7 wherein the ratio of DMXAA:docetaxel is in the range 2:1 to 1:1.

9. Use according to any of claims 1 to 8 wherein the DMXAA or pharmaceutically acceptable salt thereof and the compound selected from paclitaxel or docetaxel are to be administered concomitantly.

10. Use according to any of claims 1 to 8 wherein the DMXAA or pharmaceutically acceptable salt thereof and the compound selected from paclitaxel or docetaxel are to be administered sequentially within 2 hours of one another.

11. Use according to claim 10 wherein the compound selected from paclitaxel or docetaxel is to be administered first followed by administration within 2 hours of DMXAA or a pharmaceutically acceptable salt thereof.

12. Use according to any preceding claim wherein the pharmaceutically acceptable salt is a sodium salt.

13. A combination of DMXAA or a pharmaceutically acceptable salt thereof and a compound selected from paclitaxel or docetaxel wherein the DMXAA or pharmaceutically acceptable salt thereof and the compound selected from paclitaxel or docetaxel are present in a potentiating ratio.

14. A pharmaceutical formulation comprising a combination of DMXAA or a pharmaceutically acceptable salt thereof and a compound selected from paclitaxel or docetaxel in association with one or more pharmaceutically acceptable carriers therefor.

15. A pharmaceutical formulation according to claim 14 wherein the formulation is adapted for intravenous administration.

16. A process for the preparation of a pharmaceutical formulation which process comprises bringing into association a combination of DMXAA or a pharmaceutically acceptable salt thereof and a compound selected from paclitaxel or docetaxel with one or more pharmaceutically acceptable carriers therefor.

## Patentansprüche

1. Verwendung von 5,6-Dimethylxanthenon-4-essigsäure (DMXAA) oder eines pharmazeutisch verträglichen Salzes davon und einer unter Paclitaxel oder Docetaxel ausgewählten Verbindung bei der Herstellung eines Arzneimittels für die Behandlung von Krebs, wobei die 5,6-Dimethylxanthenon-4-essigsäure oder ein pharmazeutisch verträgliches Salz davon mit der unter Paclitaxel oder Docetaxel ausgewählten Verbindung entweder begleitend oder aufeinanderfolgend verabreicht werden soll.

2. Verwendung nach Anspruch 1, wobei die DMXAA oder das pharmazeutisch verträgliche Salz davon und die unter Paclitaxel oder Docetaxel ausgewählte Verbindung in einem potenzierenden Verhältnis vorliegen.

3. Verwendung nach Anspruch 1 oder Anspruch 2, wobei die unter Paclitaxel oder Docetaxel ausgewählte Verbindung Paclitaxel ist.

4. Verwendung nach Anspruch 3, wobei das Verhältnis DMXAA:Paclitaxel in dem Berich 220:1 bis 1:1 liegt.

5. Verwendung nach Anspruch 4, wobei das Verhältnis DMXAA:Paclitaxel in dem Bereich 2:1 bis 1:1 liegt.

6. Verwendung nach Anspruch 1 oder Anspruch 2, wobei die unter Paclitaxel oder Docetaxel ausgewählte Verbindung Docetaxel ist.

7. Verwendung nach Anspruch 6, wobei das Verhältnis von DMXAA:Docetaxel in dem Bereich 100:1 bis 1:1 liegt.

8. Verwendung nach Anspruch 7, wobei das Verhältnis von DMXAA:Docetaxel in dem Bereich 2:1 bis 1:1 liegt.

9. Verwendung nach einem der Ansprüche 1 bis 8, wobei die DMXAA oder das pharmazeutisch verträgliche Salz davon und die unter Paclitaxel oder Docetaxel ausgewählte Verbindung begleitend verabreicht werden sollen.

10. Verwendung nach einem der Ansprüche 1 bis 8, wobei die DMXAA oder das pharmazeutisch verträgliche Salz davon und die unter Paclitaxel oder Docetaxel ausgewählte Verbindung aufeinanderfolgend, eins innerhalb von 2 Stunden nach dem anderen, verabreicht werden sollen.

11. Verwendung nach Anspruch 10, wobei die unter Paclitaxel oder Docetaxel ausgewählte Verbindung zuerst verabreicht werden soll, gefolgt von der Verabreichung von DMXAA oder einem pharmazeutisch verträglichen Salz davon innerhalb von 2 Stunden.

12. Verwendung nach einem der vorherigen Ansprüche, wobei das pharmazeutisch verträgliche Salz ein Natriumsalz ist.

13. Kombination aus DMXAA oder einem pharmazeutisch verträglichen Salz davon und einer unter Paclitaxel oder Docetaxel ausgewählten Verbindung, wobei die DMXAA oder das pharmazeutisch verträgliche Salz davon und die unter Paclitaxel oder Docetaxel ausgewählte Verbindung in einem potenzierenden Verhältnis vorliegen.

14. Pharmazeutische Formulierung, die eine Kombination aus DMXAA oder einem pharmazeutisch verträglichen Salz davon und einer unter Paclitaxel oder Docetaxel ausgewählten Verbindung in Verbindung mit einem oder mehreren pharmazeutisch verträglichen Trägerstoffen dafür umfaßt.

15. Pharmazeutische Formulierung nach Anspruch 14, wobei die Formulierung für die intravenöse Verabreichung geeignet ist.

16. Verfahren zur Herstellung einer pharmazeutischen Formulierung, wobei man bei diesem Verfahren eine Kombination aus DMXAA oder einem pharmazeutisch verträglichen Salz davon und einer unter Paclitaxel oder Docetaxel ausgewählten Verbindung mit einem oder mehreren pharmazeutisch verträglichen Trägem dafür miteinander in Verbindung bringt.

## Revendications

1. Utilisation d'acide 5,6-diméthylxanthénone-4-acétique (DMXAA) ou d'un de ses sels pharmaceutiquement acceptables et d'un composé choisi entre le paclitaxel et le docétaxel dans la fabrication d'un médicament destiné au traitement du cancer, dans laquelle l'acide 5,6-diméthylxanthénone-4-acétique ou un de ses sels pharmaceutiquement acceptables est destiné à être administré conjointement ou successivement avec ledit composé choisi entre le paclitaxel et le docétaxel.

2. Utilisation suivant la revendication 1, dans laquelle le DMXAA ou un de ses sels pharmaceutiquement acceptables et le composé choisi entre le paclitaxel et le docétaxel sont présents en un rapport potentialisant.

3. Utilisation suivant la revendication 1 ou la revendication 2, dans laquelle le composé choisi entre le paclitaxel et le docétaxel est le paclitaxel.

4. Utilisation suivant la revendication 3, dans laquelle le rapport de DMXAA : paclitaxel est compris dans l'intervalle de 220:1 à 1:1.

5. Utilisation suivant la revendication 4, dans laquelle le rapport de DMXAA : paclitaxel est compris dans l'intervalle de 2:1 à 1:1.

6. Utilisation suivant la revendication 1 ou la revendication 2, dans laquelle le composé choisi entre le paclitaxel et le docétaxel est le docétaxel.

7. Utilisation suivant la revendication 6, dans laquelle le rapport de DMXAA : docétaxel est compris dans l'intervalle de 100:1 à 1:1.

8. Utilisation suivant la revendication 7, dans laquelle le rapport de DMXAA : docétaxel est compris dans l'intervalle de 2:1 à 1:1.

9. Utilisation suivant l'une quelconque des revendications 1 à 8, dans laquelle le DMXAA ou son sel pharmaceutiquement acceptable et le composé choisi entre le paclitaxel et le docétaxel sont destinés à être administrés conjointement.

10. Utilisation suivant l'une quelconque des revendications 1 à 8, dans laquelle le DMXAA ou son sel pharmaceutiquement acceptable et le composé choisi entre le paclitaxel et le docétaxel sont destinés à être administrés successivement dans la limite de 2 heures l'un de l'autre.

11. Utilisation suivant la revendication 10, dans laquelle le composé choisi entre le paclitaxel et le docétaxel est destiné à être administré en premier, avec ensuite l'administration, dans la limite de 2 heures, du DMXAA ou d'un de sels pharmaceutiquement acceptables.

12. Utilisation suivant l'une quelconque des revendications précédentes, dans laquelle le sel pharmaceutiquement acceptable est un sel de sodium.

13. Association de DMXAA ou d'un de ses sels pharmaceutiquement acceptables et d'un composé choisi entre le paclitaxel et le docétaxel, dans laquelle le DMXAA ou son sel pharmaceutiquement acceptable et le composé choisi entre le paclitaxel et le docétaxel sont présents en un rapport potentialisant.

14. Formulation pharmaceutique comprenant une association de DMXAA ou d'un de ses sels pharmaceutiquement acceptables et d'un composé choisi entre le paclitaxel et le docétaxel, en association avec un ou plusieurs supports pharmaceutiquement acceptables.

15. Formulation pharmaceutique suivant la revendication 14, ladite formulation étant adaptée à l'administration intraveineuse.

16. Procédé pour la préparation d'une formulation pharmaceutique, procédé qui comprend l'étape consistant à associer une association de DMXAA ou d'un de ses sels pharmaceutiquement acceptables et d'un composé choisi entre le paclitaxel et le docétaxel avec un ou plusieurs supports pharmaceutiquement acceptables.
